# EUROPEAN PATENT SPECIFICATION

(11) **EP 3 239 709 B1**
(45) Date of publication and mention of the grant of the patent: **05.06.2019**
(21) Application number: 17167152.2
(22) Date of filing: 19.04.2017
(51) Int. Cl.: G01N 33/52, G01N 33/66

(54) **METHOD OF ANALYZING GLYCATED PROTEIN; USE OF ANALYSIS REAGENT, ANALYSIS KIT, AND TEST PIECE FOR ANALYSIS**
VERFAHREN ZUR ANALYSE VON GLYKIERTEM PROTEIN; VERWENDUNG VON ANALYSEREAGENS, ANALYSE-KIT UND TESTSTÜCK ZUR ANALYSE
PROCÉDÉ D'ANALYSE D'UNE PROTÉINE GLYCOSYLÉE; UTILISATION D'UN RÉACTIF D'ANALYSE, D'UN KIT D'ANALYSE ET D'UNE PIÈCE D'ESSAI POUR ANALYSE

(30) Priority: 25.04.2016 JP 2016087040; 17.04.2017 JP 2017081508
(43) Date of publication of application: 01.11.2017
(73) Proprietor: ARKRAY, Inc., Minami-ku Kyoto-shi, Kyoto 601-8045 (JP)
(72) Inventor: NAKAMURA, Tsutomu, Kyoto-shi, Kyoto 602-0008 (JP); HAMA, Takashi, Kyoto-shi, Kyoto 602-0008 (JP)
(74) Representative: Dehns

(56) References cited:
- EP-A1- 0 636 885
- EP-A1- 1 329 721
- US-A1- 2009 169 430
- US-A1- 2016 084 850
- Ref ET AL: "FT-F98881 Water Soluble Tetrazolium Salts (WSTs)", Interchim Life Sciences, 1 January 1993 (1993-01-01), pages 1/3-3/3, XP055385052, Retrieved from the Internet: URL:http://www.interchim.fr/ft/F/F98881.pd f

## Description

### BACKGROUND

### Technical Field

The present disclosure relates to a method of analyzing a glycated protein, an analysis reagent, an analysis kit, and a test piece for analysis.

### Related Art

Blood glycated proteins have been utilized as an index of short-term glycemic control. Blood proteins are bound with the aldehyde group of glucose at an amino group of their *N*-terminals or side chains and further subjected to Amadori rearrangement, as a result of which glycated proteins are generated as Amadori compounds. These glycated proteins are also referred to as "fructosamine" since their side chains assume a fructose structure. Fructosamine is a generic term for glycated proteins existing in blood.

Glycated proteins are reducing substances and are thus measured by measuring a degree of a color development of a substrate (e.g., 2-(4-indophenyl)-3-(4-nitrophenyl)-5-phenyl-2*H*-tetrazolium chloride (INT)), which develops a color by reacting with a reducing substance. However, since such a substrate also measures a blood reducing substance (e.g., uric acid) other than glycated proteins, there is a problem that the use of such a substrate does not yield an accurate value of a blood glycated protein.

In view of this, the following methods have been reported for the avoidance of the effects of a reducing substance other than glycated proteins. A first method is a method in which, for a sample, the amount of change in absorbance is measured using a first reagent that reacts with blood reducing substances including glycated proteins while the amount of change in absorbance is also measured using a second reagent that reacts with blood reducing substances other than glycated proteins, and the net change in absorbance of glycated proteins is determined by subtracting the latter value from the former value (Japanese Patent Application Laid-Open (JP-A) No. H06-308129). A second method is a method for avoiding the effects of a blood reducing substance other than glycated proteins by a pretreatment of a sample with uricase which breaks down uric acid, glycosaminoglycan which is an insoluble carrier, or the like (JP-A No. H07-151761 and JP-A No. H08-226920).

However, the first method requires two reaction systems where different reagents are used for a sample (double reaction system), and is thus laborious and costly. Further, in the second method, the use of uricase has a problem of causing turbidity in a reaction solution and thereby affecting the absorbance measurement, and the use of glycosaminoglycan which is an insoluble carrier requires immobilization thereof, making the operations for washing, quenching and the like complicated.

### SUMMARY OF THE INVENTION

### Problems to be Solved by the Invention

In view of the above, an object of the disclosure is to provide a method by which a glycated protein can be analyzed more simply and accurately.

### Means for Solving the Problems

The method of analyzing a glycated protein according to the disclosure is characterized by including: reacting a color developer represented by the following Formula (1) with a biological sample; and measuring a color reaction of the color developer: 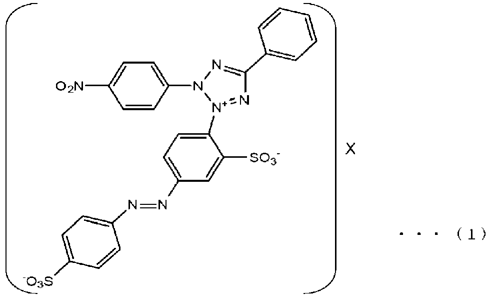 (wherein, X is an alkali metal ion).

The reagent for analyzing a glycated protein according to the disclosure includes a color developer represented by the following Formula (1) and is used in the analysis method of the disclosure: 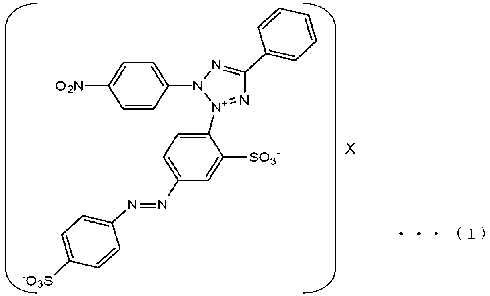 (wherein, X is an alkali metal ion).

The kit for analyzing a glycated protein according to the disclosure includes the analysis reagent of the disclosure and is used in the analysis method of the disclosure.

The test piece for analyzing a glycated protein according to the disclosure includes a substrate and is characterized in that:
the substrate includes a sample supply region and a reaction region,
the sample supply region and the reaction region are connected by a flow channel,
the analysis reagent of the disclosure is arranged in the reaction region, and
the reaction region in the substrate is composed of a transparent member.

### Effects of the Invention

According to the disclosure, by using a color developer represented by Formula (1), a glycated protein can be analyzed more simply and accurately.

### BRIEF DESCRIPTION OF THE DRAWINGS

FIG. 1 shows one example of the analysis test piece of the disclosure, wherein (A) is a plane view of the analysis test piece, and (B) is a cross-sectional view taken along the I-I direction of the analysis test piece shown in (A).
   (Description of Symbols)
   1: Analysis test piece
   2: Substrate
   2a: Upper substrate
   2b: Lower substrate
   3: Sample supply region
   3a, 5c: Opening
   3b: First space
   4: Second space
   5a, 5b: Flow channel
FIG. 2 provides graphs showing the results of measuring the absorbance in Example 1 of the disclosure;
FIG. 3 provides graphs showing the results of measuring the absorbance in Comparative Example 1 of the disclosure;
FIG. 4 provides graphs showing the results of measuring the absorbance in Comparative Example 2 of the disclosure;
FIG. 5A provides graphs showing the results of measuring the absorbance in Example 2 of the disclosure;
FIG. 5B provides graphs showing the results of measuring the absorbance in Example 2 of the disclosure;
FIG. 6A provides graphs showing the results of measuring the absorbance in Example 2 of the disclosure; and
FIG. 6B provides graphs showing the results of measuring the absorbance in Example 2 of the disclosure.

### DETAILED DESCRIPTION OF THE INVENTION

### Mode for Carrying Out the Invention

In the method of analyzing a glycated protein according to the disclosure, for example, the biological sample is a blood sample.

In the method of analyzing a glycated protein according to the disclosure, for example, the blood sample is a serum sample or a plasma sample.

In the method of analyzing a glycated protein according to the disclosure, for example, in the reaction, the color developer and the biological sample are allowed to react with each other by adding the biological sample in a liquid state to the color developer in a dry state.

In the test piece for analyzing a glycated protein according to the disclosure, for example,
the substrate is a layered body including an upper substrate and a lower substrate,
the inside of the layered body includes a first space, a second space, and the flow channel connecting the first space and the second space,
the upper substrate includes an opening at a position corresponding to the first space,
the opening of the upper substrate and the first space form the sample supply region,
the opening is a sample inlet,
the second space is the reaction region, and
the analysis reagent of the disclosure is arranged in at least one of a region of the upper substrate or a region of the lower substrate, which regions correspond to the second space.

### (Method of Analyzing Glycated Protein in Biological Sample)

As described above, the method of analyzing a glycated protein according to the disclosure (hereinafter, referred to as "the analysis method of the disclosure") is characterized by including: the reaction step of allowing a color developer represented by the following Formula (1) and a biological sample to react with each other; and the measurement step of measuring a color reaction of the color developer: 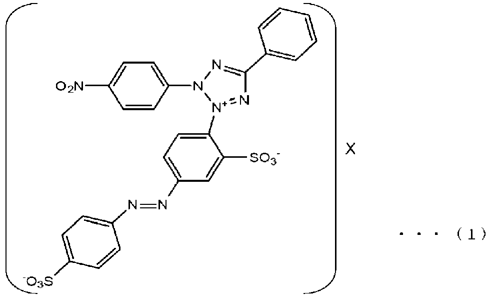 (wherein, X is an alkali metal ion).

According to the disclosure, by using the color developer as a tetrazolium salt, reaction between the color developer and a glycated protein contained in a biological sample can be performed while avoiding the effects of uric acid that is a reducing substance, although the reason therefor is not clear. Thus, for example, even in a case where uric acid is contained in a biological sample along with a glycated protein, an increase in apparent coloration due to reaction with uric acid can be inhibited, and this enables to measure the degree of coloration that corresponds to the amount of the glycated protein contained in the sample. Further, the disclosure does not require such a measurement in a double reaction system using two or more reagents as described above for the purpose of, for example, eliminating the effects of uric acid, and the measurement can be sufficiently performed by at least a single reaction system alone (However, there is no intention to exclude two reagent systems from the invention). Moreover, in the disclosure, for example, it is also not required to use uricase or an insoluble carrier as described above for avoidance of the effects of uric acid in combination with the color developer.

Glycated proteins in blood are Amadori compounds which are generated by glycation of proteins contained in blood by blood glucose during circulation in the body, and their reducing powers are measured using the color developer. That is, glycated proteins that can be measured using the color developer include glycation products of all proteins that can be glycated in blood. Examples of such glycated proteins include glycated albumin, glycated hemoglobin, and glycated globulin.

A subject of the method of analyzing a glycated protein, the analysis kit, and the test peace for analysis of the present disclosure includes not only the total amount of glycated proteins (i.e. fructosamine), but also individual glycated proteins or a certain group of glycated proteins. The disclosure encompasses a measurement of a specific glycated protein, for example, by excluding glycated proteins, that is not subjected to be measured, from a sample through a pre-treatment, or by blocking glycated proteins, that is not subjected to be measured, in a sample to avoid a reaction with the color developer.

The analysis method of the disclosure iis characterized in that the color developer and a biological sample are allowed to react in the reaction step, and other steps and conditions are not particularly restricted.

The analysis method of the disclosure may be used for, for example, qualitative analysis where the presence or absence of a glycated protein is analyzed, or quantitative analysis where the amount of a glycated protein is analyzed. The analysis method of the disclosure can also be regarded as, for example, a method of measuring a glycated protein.

In the color developer, as described above, X is an alkali metal ion. Examples of the alkali metal ion include a lithium ion, a sodium ion, a potassium ion, a rubidium ion, a cesium ion, and a francium ion, among which a sodium ion (Na⁺) is preferred.

Specific examples of the color developer include 2-(4-nitrophenyl)-5-phenyl-3-[4-(4-sulfophenylazo)-2-sulfophenyl]-2*H*-tetrazolium sodium salt (also referred to as "WST-9"), which is represented by the following Formula (2).

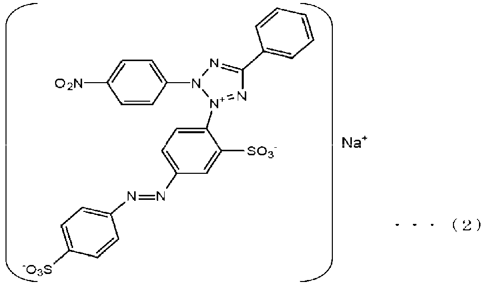

In the reaction step, the addition ratio of the color developer with respect to the biological sample is not particularly restricted. The color developer is added in an amount of, for example, from 1 to 20 nmol, from 1 to 10 nmol, or from 1 to 7.5 nmol, with respect to 1 µL of the biological sample.

In the analysis method of the disclosure, the biological sample is not particularly restricted, and examples thereof include samples in which a glycated protein is expected to be present, which may be a glycated protein-containing sample or a glycated protein-free sample. The biological sample may be, for example, a blood sample, examples of which include erythrocytes, whole blood, serum, and plasma. The blood sample may also be, for example, a hemolyzed sample when it contains hemocytes.

In the reaction step, the reaction system of the color developer and the biological sample may further includes, for example, other additive components. Examples of the additive components include buffers, surfactants, excipients, and enzymes.

The pH of the reaction system is not particularly restricted and can be deteremined as appropriate in accordance with, for example, the color developer. The pH of the reaction system is, for example, from 9.5 to 11.5 and, when the color developer is WST-9, the pH of the reaction system is, for example, from 9.5 to 11.5. The pH of the reaction system can be adjusted with a buffer or the like. As the buffer, a conventionally known buffer can be used as appropriate, and examples thereof include CHES (N-Cyclohexyl-2-aminoethanesulfonic acid) buffers, sodium carbonate buffers.

Examples of the enzymes include oxidoreductases, such as ascorbate oxidase.

Examples of the surfactants include polyoxyethylene sorbitan alkyl esters (e.g., TWEEN-type surfactants), sodium taurodeoxycholate, 3-[(3-cholamidopropyl)dimethylammonio]propane sulfonate, lauric acid amide propyl betaine, octylphenol ethoxylate, dodecyldimethyl(3-sulfopropyl)ammonium hydroxide inner salt, polyoxyethylene lauryl ethers, and sodium cholate. Examples of the TWEEN-type surfactants include TWEEN 20 (trade name, manufactured by Nacalai Tesque, Inc.); examples of sodium taurodeoxycholate include taurodeoxycholic acid sodium salt (trade name, manufactured by Nacalai Tesque, Inc.); examples of 3-[(3-cholamidopropyl)dimethylammonio]propane sulfonate include CHAPS (trade name, manufactured by Dojindo Laboratories Inc.); examples of lauric acid amide propyl betaine include AMPHITOL 20AB (trade name, manufactured by Kao Corporation); examples of octylphenol ethoxylate include TRITON X-100 (trade name, manufactured by Nacalai Tesque, Inc.); examples of dodecyldimethyl(3-sulfopropyl)ammonium hydroxide inner salt include caprylyl sulfobetaine (trade name, manufactured by Tokyo Chemical Industry Co., Ltd.); and examples of the polyoxyethylene lauryl ethers include BRIG 35 (trade name, manufactured by Wako Pure Chemical Industries, Ltd.).

Examples of the excipients include sorbitol, sucrose, and xylitol.

In the analysis method of the disclosure, as described above, neither uricase nor insoluble carrier (e.g., glycosaminoglycan) is required. Thus, the analysis method of the disclosure is performed, for example, in the absence of uricase and, specifically, it is preferred that the analysis method of the disclosure is performed in a state where external uricase that is not derived from the biological sample to be analyzed is not added. Further, it is preferred that the analysis method of the disclosure is performed in the absence of the insoluble carrier (e.g., glycosaminoglycan).

In the analysis method of the disclosure, the reaction between the color developer and the biological sample in the reaction step may be performed, for example, in a wet system or in a dry system. The former is, for example, a reaction performed in a liquid containing the color developer, whereas the latter is, for example, a reaction performed on a test piece on which the color developer is arranged. The analysis method of the disclosure is particularly suitable for the latter dry system since, as described above, the analysis method is capable of performing measurements in a single reaction system and requires neither uricase nor insoluble carrier (e.g., glycosaminoglycan).

For the analysis method of the disclosure, a mode of the dry system will now be exemplified. It is noted here, however, that the disclosure is not restricted thereto. When the analysis method of the disclosure is a dry system, for example, the below-described analysis test piece of the disclosure can be used.

When the analysis method of the disclosure is a dry system, for example, in the reaction step, the biological sample in a liquid state and a dry reagent containing the color developer are allowed to react with each other by adding the biological sample to the dry reagent. According to this mode, for example, the dry reagent and the biological sample are mixed by adding the latter to the former, whereby the color developer contained in the dry reagent and the biological sample are allowed to react with each other.

The dry reagent used in the reaction step may be, for example, a single dry reagent containing the color developer, or a combination of two or more dry reagents. For example, in cases where two or more dry reagents are used in combination, before allowing a dry reagent containing the color developer and a sample to react with each other, other dry reagent(s) and the sample may be brought into contact to perform pH adjustment or a pretreatment such as enzyme reaction.

In cases where a single dry reagent is used as described above, the dry reagent contains the color developer and may further contain the above-described additive component(s). The dry reagent can be obtained by, for example, preparing a liquid reagent that contains the color developer and arbitrary additive component(s) and subsequently drying the liquid reagent. In the liquid reagent, for example, it is preferred that the pH is set at a level appropriate for the color developer, and the pH can be adjusted with a buffer or the like. The pH is, for example, from 9.5 to 11.5, and a sodium carbonate buffer is preferred as the buffer.

In cases where two or more dry reagents are used in combination as described above, at least one of the dry reagents may include the color developer. For example, when a first dry reagent and a second dry reagent are used in combination as the two dry reagents, the first dry reagent contains at least the color developer and at least one of the above-described additive components, and the second dry reagent contains at least one of the above-described additive components.

It is preferred that the first dry reagent contains, for example, at least one of the above-described buffers, surfactants, excipients and enzymes (e.g., ascorbate oxidase) as the additive component. The first dry reagent can be obtained by, for example, preparing a first liquid reagent that contains the color developer and the additive component(s) and subsequently drying the first liquid reagent. In the first dry reagent, the buffer is, for example, the buffer that is contained in the first liquid reagent and dried. In the first liquid reagent, the pH can be set at a level appropriate for the color developer, which is, for example, from 5.5 to 6.5, or 6. A stability of a component in the first dry reagent can be improved by setting the pH within the range of from 5.5 to 6.5. The pH can be adjusted with a buffer or the like and, for example, a MES-KOH buffer can be used as the buffer.

In cases where two or more dry reagents are used in combination as described above, the buffer can be incorporated into the two or more dry reagents such that the reaction system has a pH appropriate for the color developer, which is, for example, from 9.5 to 11.5. Those of ordinary skill in the art can select the type and the concentration of the buffer in accordance with the intended pH.

In the first dry reagent, from the viewpoint of improving lubricating property in a dry state, examples of the surfactants include polyoxyethylene sorbitan alkyl esters (e.g., TWEEN-type surfactants), sodium taurodeoxycholate, *n*-octanoyl-*N*-methyl-D-glucamine, octylphenol ethoxylate, and 3-[(3-cholamidopropyl)dimethylammonio]propane sulfonate. Examples of the TWEEN-type surfactants include TWEEN 20 (trade name, manufactured by Nacalai Tesque, Inc.); examples of sodium taurodeoxycholate include taurodeoxycholic acid sodium salt (trade name, manufactured by Nacalai Tesque, Inc.); examples of *n*-octanoyl-*N*-methyl-D-glucamine include MEGA-8 (trade name, manufactured by Dojindo Laboratories Inc.); examples of octylphenol ethoxylate include TRITON X-100 (trade name, manufactured by Nacalai Tesque, Inc.); and examples of 3-[(3-cholamidopropyl)dimethylammonio]propane sulfonate include CHAPS (trade name, manufactured by Dojindo Laboratories Inc.). In the first reagent, examples of the excipients include sorbitol, sucrose, and xylitol.

It is preferred that the second dry reagent contains, for example, at least one of the above-described buffers, surfactants and excipients as the additive component. The second dry reagent can be obtained in the same manner as the first dry reagent by, for example, preparing a second liquid reagent that contains the additive component(s) and subsequently drying the second liquid reagent. In the second dry reagent, the buffer is, for example, the buffer that is contained in the second liquid reagent and dried. The pH of the second liquid reagent is not particularly restricted, and it is, for example, from 9.5 to 11.5. The pH can be adjusted with a buffer or the like and, for example, a sodium carbonate buffer can be used as the buffer.

In the second dry reagent, from the viewpoint of improving lubricating property in a dry state and specificity, examples of the surfactants include *n*-octanoyl-*N*-methyl-D-glucamine, lauric acid amide propyl betaine, sodium taurodeoxycholate, sodium cholate, 3-[(3-cholamidopropyl)dimethylammonio]propane sulfonate, polyoxyethylene lauryl ethers, dodecyldimethyl(3-sulfopropyl)ammonium hydroxide inner salt, and polyoxyethylene sorbitan alkyl esters (e.g., TWEEN-type surfactants). Examples of *n*-octanoyl-*N*-methyl-D-glucamine include MEGA-8 (trade name, manufactured by Dojindo Laboratories Inc.); examples of lauric acid amide propyl betaine include AMPHITOL 20AB (trade name, manufactured by Kao Corporation); examples of sodium taurodeoxycholate include taurodeoxycholic acid sodium salt (trade name, manufactured by Nacalai Tesque, Inc.); examples of 3-[(3-cholamidopropyl)dimethylammonio]propane sulfonate include CHAPS (trade name, manufactured by Dojindo Laboratories Inc.); examples of the polyoxyethylene lauryl ethers include BRIG 35 (trade name, manufactured by Wako Pure Chemical Industries, Ltd.); examples of dodecyldimethyl(3-sulfopropyl)ammonium hydroxide inner salt include caprylyl sulfobetaine (trade name, manufactured by Tokyo Chemical Industry Co., Ltd.); and examples of the TWEEN-type surfactants include TWEEN 20 (trade name, manufactured by Nacalai Tesque, Inc.). In the second dry reagent, examples of the excipients include sorbitol, sucrose, and xylitol.

It is preferred that the dry reagent is arranged on a carrier or the like, and examples of the carrier include the below-described substrate. When two or more dry reagents are used in combination as described above, the two or more dry reagents may be arranged on the same part of the substrate, or on different parts of the substrate. In the latter case, it is preferred that the two or more dry reagents are arranged in such a manner that addition of the biological substance thereto allows the biological sample to be mixed with all of the dry reagents.

In the measurement step, the measurement of color reaction means, for example, the measurement of the coloration of the color developer caused by the color reaction. The coloration can be measured in terms of, for example, an optical signal. The optical signal is not particularly restricted, and examples thereof include absorbance, reflectance, and transmittance, among which absorbance is preferred.

In the measurement step, it is preferred that the measurement of color reaction is performed, for example, in a wavelength range where the coloration of the color developer shows a peak. The wavelength range can be decided as appropriate in accordance with, for example, the type of the color developer. When the color developer is WST-9, the measurement wavelength is in a range of, for example, from 400 to 700 nm, from 450 to 660 nm, or from 500 to 610 nm.

In the measurement method of the disclosure, it can be said that a value of the color reaction measured in the measurement step is in a relative relationship with a glycated protein contained in the biological sample. Therefore, the measurement results obtained in the measurement step may be directly taken as values which indirectly show the amount or concentration of the glycated protein in the biological sample, or the amount or concentration of the glycated protein in the biological sample may be calculated from the measurement results obtained in the measurement step. In the latter case, the calculation can be made based on, for example, the correlation between the results of measuring the color reaction (e.g., measured values of an optical signal) and the concentration of the glycated protein in the biological sample.

For the measurement of color reaction, for example, a dry-type clinical chemistry analyzer (trade name: SPOTCHEM EZ, SPOTCHEM D-CONCEPT, or SPOTCHEM II glycated protein single-item analyzer; manufactured by ARKRAY Inc.) can be used. Such an analyzer can be utilized, for example, when the above-described analysis test piece is used in a dry system.

When the analysis method of the disclosure is a wet system, the measurement can be performed in the same manner by applying the above-described method and conditions of the dry system, except that the liquid reagent is used in place of the dry reagent.

### (Reagent for Analyzing Glycated Protein)

The reagent for analyzing a glycated protein according to the disclosure (hereinafter, referred to as "the analysis reagent of the disclosure") is characterized in that it includes the above-described color developer and is used in the analysis method of the disclosure. The characteristics feature of the analysis reagent of the disclosure is the inclusion of the color developer, and other constitutions and conditions are not particularly restricted. The analysis reagent of the disclosure thus also encompasses, for example, a test piece whose structure is different from that of the below-described test piece for analyzing a glycated protein, as long as the test piece contains the color developer. The analysis reagent of the disclosure is, for example, the above-described dry reagent or liquid reagent used in the analysis method of the disclosure, and the above descriptions can thus be applied thereto.

### (Kit for Analyzing Glycated Protein)

The kit for analyzing a glycated protein in a biological sample according to the disclosure (hereinafter, referred to as "the analysis kit of the disclosure") is characterized in that it includes the analysis reagent of the disclosure and is used in the analysis method of the disclosure. The characteristics feature of the analysis kit of the disclosure is the inclusion of the analysis reagent, and other constitutions and conditions are not particularly restricted. The analysis kit of the disclosure may further includes, for example, an instruction manual. For the analysis kit of the disclosure, for example, the above descriptions on the dry reagent and the liquid reagent in the analysis method of the disclosure can be applied.

### (Test Piece for Analyzing Glycated Protein)

The test piece for analyzing a glycated protein according to the disclosure (hereinafter, referred to as "the analysis test piece of the disclosure") includes a substrate and is characterized in that:
the substrate includes a sample supply region and a reaction region;
the sample supply region and the reaction region are connected by a flow channel;
the analysis reagent of the disclosure is arranged in the reaction region; and
the reaction region in the substrate is composed of a transparent member.

The characteristics feature of the analysis test piece of the disclosure is that the analysis reagent of the disclosure is arranged in the reaction region, and other constitutions and conditions are not particularly restricted.

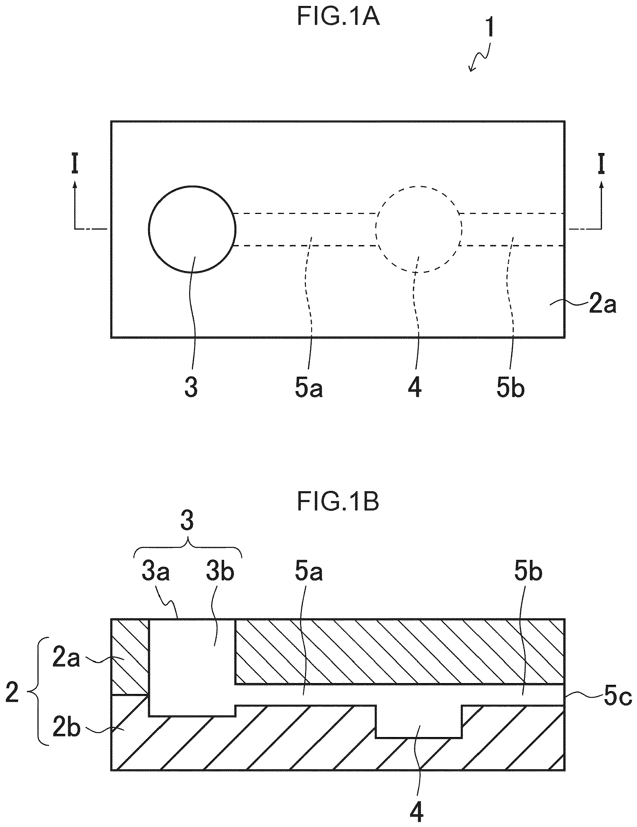

FIG. 1 shows one example of the analysis test piece of the disclosure. FIG. 1(A) is a plan view of an analysis test piece 1, and FIG. 1(B) is a cross-sectional view taken along the I-I direction of FIG. 1(A).

As shown in FIGs. 1(A) and (B), the analysis test piece 1includes: a substrate 2 in which an upper substrate 2a and a lower substrate 2b are disposed in layers; and the analysis reagent of the disclosure. The inside of the substrate 2 includes a first space 3b, a second space 4, and a flow channel 5a connecting the first space 3b and the second space 4. The upper substrate 2a includes an opening 3a at a position corresponding to the first space 3b. The opening 3a of the upper substrate 2a and the first space 3b form a sample supply region 3, and the opening 3a is a sample inlet. The second space 4 is a reaction region 4, and a region of the substrate 2 corresponding to the reaction region 4 also serves as a detection region. Further, the substrate 2 includes an opening 5c and a flow channel 5b connecting the second space 4 and the opening 5c, and the opening 5c serves as an air vent.

In the analysis test piece 1, the analysis reagent of the disclosure is, for example, the above-described dry reagent. The part for arranging the analysis reagent is, for example, the reaction region 4, and the analysis reagent is arranged in at least one of a region of the upper substrate 2a and a region of the lower substrate 2b, which regions correspond to the second space 4. In cases where the analysis reagent of the disclosure is composed of two or more dry reagents as described above, for example, it is preferred that the dry reagents are each arranged in the region of the upper substrate 2a and the region of the lower substrate 2b, which regions correspond to the second space 4.

The material of the substrate 2 is not particularly restricted, and examples thereof include glass and resins. In the substrate 2 (the upper substrate 2a and the lower substrate 2b), specifically, it is preferred that the part corresponding to the reaction region is formed by a transparent member since the part also serves as the detection region. Examples of the transparent member include members made of PS (polystyrene), PET (polyethylene terephthalate), PC (polycarbonate), PMMA (polyacrylic resin) or the like. The material of the upper substrate 2a and that of the lower substrate 2b may be the same or different.

In the analysis test piece of the disclosure, for example, a method of arranging the analysis reagent of the disclosure is not particularly restricted, and the above descriptions on the dry system in the analysis method of the disclosure can be applied thereto. That is, the dry reagent(s) can be arranged by spotting and then drying the liquid reagent(s) of the disclosure at a desired position(s) of the substrate.

The analysis test piece of the disclosure can be used, for example, in the following manner. First, the biological sample is supplied to the first space 3b via the opening 3a. The biological sample passes through the flow channel 5a from the first space 3b and reaches the second space 4 (reaction region). In the second space 4, the biological sample and the analysis reagent of the disclosure arranged therein are mixed, and the biological sample and the analysis reagent are thereby allowed to react with each other, causing a color reaction of the color developer to occur. The resulting coloration is measured from the upper substrate 2a side corresponding to the reaction region 4, or from the lower substrate 2b side corresponding to the reaction region 4.

The size of the analysis test piece of the disclosure is not particularly restricted and, for example, the following conditions can be exemplified. It is noted here that, hereinafter, the term "longitudinal direction" refers to the direction that runs from the sample supply region to the reaction region, the term "width direction" refers to the direction perpendicular to the longitudinal direction, and the term "thickness direction" refers to the direction that runs from the upper substrate to the lower substrate.
Length in the longitudinal direction (total length): 5 to 20 mm (10 mm)
Length in the width direction (width): 3 to 10 mm (5.1 mm)
Length in the thickness direction (thickness): 0.25 to 3 mm (1 mm)
Size of the sample supply region 3:
   1 to 8 mm (2.5 mm) in diameter, 0.1 to 1.0 mm (0.25 mm) in depth
Size of the reaction region 4:
   1 to 8 mm (2.5 mm) in diameter, 0.1 to 1.0 mm (0.25 mm) in depth Length of the flow channel 5a in the longitudinal direction: 0.2 to 10 mm

### EXAMPLES

Examples of the disclosure will now be described. However, the disclosure is not restricted to the below-described Examples. It is noted here that, unless otherwise specified, commercially available reagents were used based on their protocols.

### [Example 1]

For the method of analyzing a glycated protein according to the disclosure, it was confirmed that the glycated protein concentration can be measured without any effect of uric acid that is a reducing substance in blood, by measurement of fructosamine.

### (1) Samples

A serum 1 (without addition of uric acid (-)) having a fructosamine concentration of 198.5 µmol/L and a serum 2 (without addition of uric acid (-)) having a fructosamine concentration of 503 µmol/L, as well as a serum 1 (with addition of uric acid (+)) and a serum 2 (with addition of uric acid (+)) which were obtained by adding uric acid to the serum 1 (without addition of uric acid (-)) and the serum 2 (without addition of uric acid (-)) at a concentration of 20 mg/100 mL, respectively, were prepared as serum samples.

### (2) Preparation of Analysis Test Piece

Using first and second liquid reagents having the following formulations, an analysis test piece of this Example shown in FIG. 1 was prepared.

**<First Reagent>**

| | |
|---|---|
| Sodium carbonate buffer (pH 11.0) | 500 mmol/L |
| (manufactured by Wako Pure Chemical Industries, Ltd.) | |
| Sorbitol | 15% (w/V) |
| Lauric acid amide propyl betaine | 0.005% (w/V) |
| (trade name: AMPHITOL 20AB, manufactured by Kao Corporation) | |
| *n*-octanoyl-*N*-methyl-D-glucamine | 0.25% (w/V) |
| (trade name: MEGA-8, manufactured by Dojindo Laboratories Inc.) | |

**<Second Reagent>**

| | |
|---|---|
| MES-KOH buffer (pH 6.0) | 10 mmol/L |
| (manufactured by Nacalai Tesque, Inc.) | |
| Color developer | 5 mmol/L |
| (trade name: WST-9, manufactured by Dojindo Laboratories Inc.) | |
| Sorbitol | 10% (w/v) |
| Sucrose | 5% (w/v) |
| TWEEN-type surfactant | 0.02% (w/v) |
| (trade name: TWEEN 20, manufactured by Nacalai Tesque, Inc.) | |
| Sodium taurodeoxycholate | 0.5% (w/v) |
| (trade name: Sodium taurodeoxycholate, manufactured by Nacalai Tesque, Inc.) | |
| Ascorbate oxidase | 200 U/mL |
| (trade name: Ascorbate oxidase, manufactured by Roche Diagnostics K.K.) | |

First, 1.5 µL of the second reagent was spotted in a region that is on a surface of upper substrate 2a to be faced to lower substrate 2b and that corresponds to the reaction region 4. Further, 1.5 µL of the first reagent was spotted in a region that is on a surface of lower substrate 2b to be faced to lower substrate 2a and that corresponds to the reaction region 4. Then, upper substrate 2a and lower substrate 2b were subjected to a 20-hour drying treatment under the conditions of 20°C and 1% relative humidity. As a result of this drying treatment, the second dry reagent and the first dry reagent were arranged on the upper substrate 2a and the lower substrate 2b, respectively. Thereafter, the upper substrate 2a and the lower substrate 2b were disposed and pasted on each other to prepare the analysis test piece 1 shown in FIG. 1.

The size and the material of the analysis test piece 1 were as follows.
Material of the upper and lower substrates: polystyrene (transparent member)
Length in the longitudinal direction (total length): 10 mm
Length in the width direction (width): 5.1 mm
Length in the thickness direction (thickness): 1.2 mm
Size of the sample supply region 3: 2.5 mm in diameter × 0.25 mm in depth
Size of the reaction region 4: 2.5 mm in diameter × 0.25 mm in depth
Length of the flow channel 5a in the longitudinal direction: 1 mm

### (3) Measurement of Fructosamine Concentration

The above-prepared serum samples were each added to the sample supply region 3 of the analysis test piece 1. The analysis test piece 1 was subsequently set in a dry-type clinical chemistry analyzer (trade name: SPOTCHEM EZ, manufactured by ARKRAY Inc.). Then, with the time point of 480 seconds after the addition of each sample being defined as the measurement starting point, the absorbance of the reaction region 4 of the analysis test piece 1 was measured over time at a measurement wavelength of 550 nm. Further, the change in absorbance per unit time was determined based on the difference (Ay - Ax) between the absorbance at a measurement time x (Ax) and the absorbance at a measurement time y (Ay). It is noted here that, in those cases where the absorbance was measured twice under the same conditions, the average value of the difference was determined as the change in absorbance per unit time.

As Comparative Example 1, another analysis test piece 1 was prepared in the same manner as described above, except that 2-(4-indophenyl)-3-(4-nitrophenyl)-5-phenyl-2*H*-tetrazolium chloride (INT, manufactured by Dojindo Laboratories Inc.) was used in place of the color developer WST-9. Further, as Comparative Example 2, yet another analysis test piece 1 was prepared in the same manner as described above, except that 2-(2-methoxy-4-nitrophenyl)-3-(4-nitrophenyl)-5-(2,4-disulfophenyl)-2*H*-tetrazolium (WST-8, manufactured by Dojindo Laboratories Inc.) was used in place of the color developer WST-9. For Comparative Example 1, a serum 1 (without addition of uric acid (-)) having a fructosamine concentration of 198.5 µmol/L and a serum 3 (without addition of uric acid (-)) having a fructosamine concentration of 786 µmol/L, as well as a serum 1 (with addition of uric acid (+)) and a serum 3 (with addition of uric acid (+)) which were obtained by adding uric acid to the serum 1 (without addition of uric acid (-)) and the serum 3 (without addition of uric acid (-)) at a concentration of 20 mg/100 mL, respectively, were prepared as serum samples. For Comparative Example 2, the same serums as in the above (1) were prepared as serum samples. Then, using the respective analysis test pieces 1 of Comparative Examples 1 and 2 and the above-prepared serum samples, the measurement of the absorbance was performed in the same manner as described above. For the analysis test piece 1 of Comparative Example 1 and the analysis test piece 1 of Comparative Example 2, the measurement wavelength was set to be 550 nm and 450 nm, respectively.

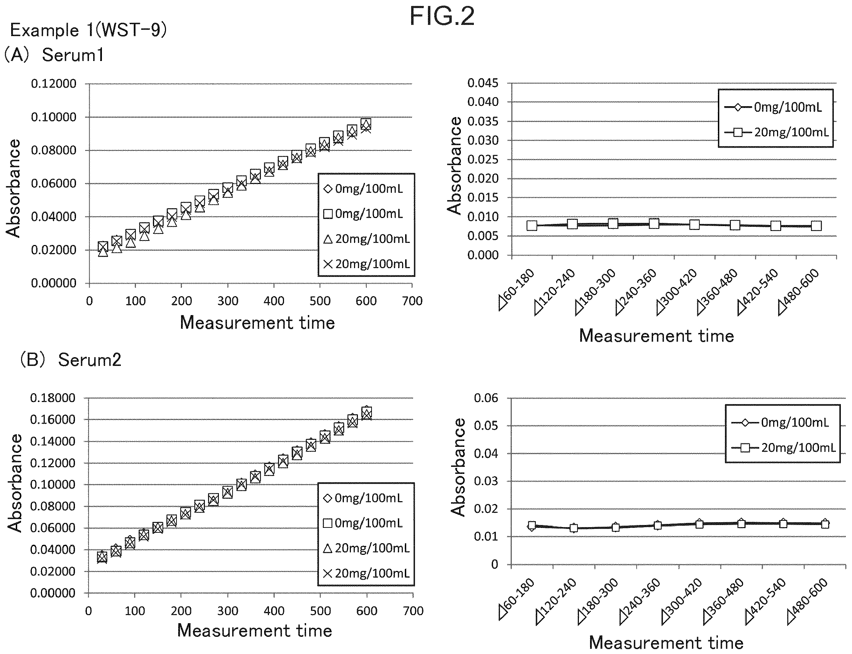

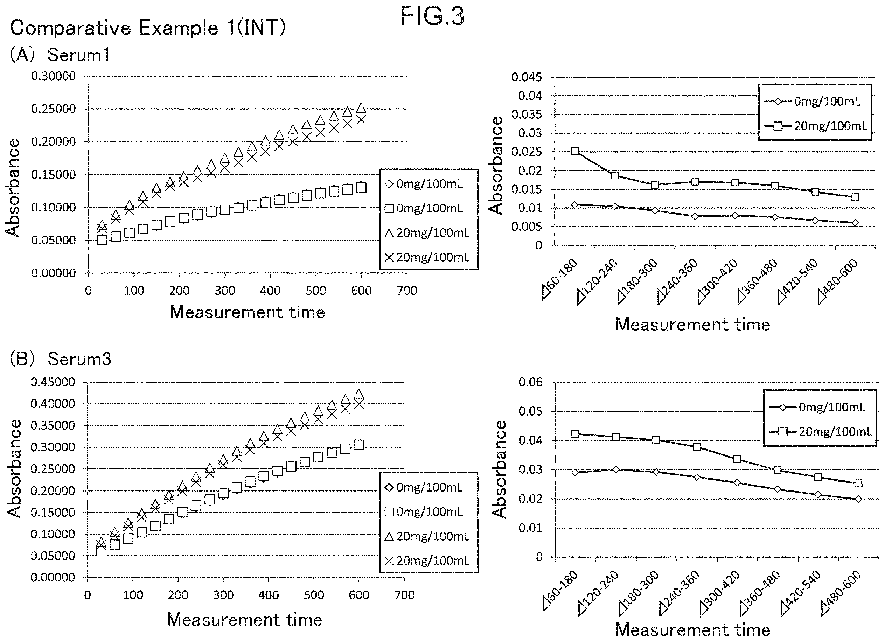

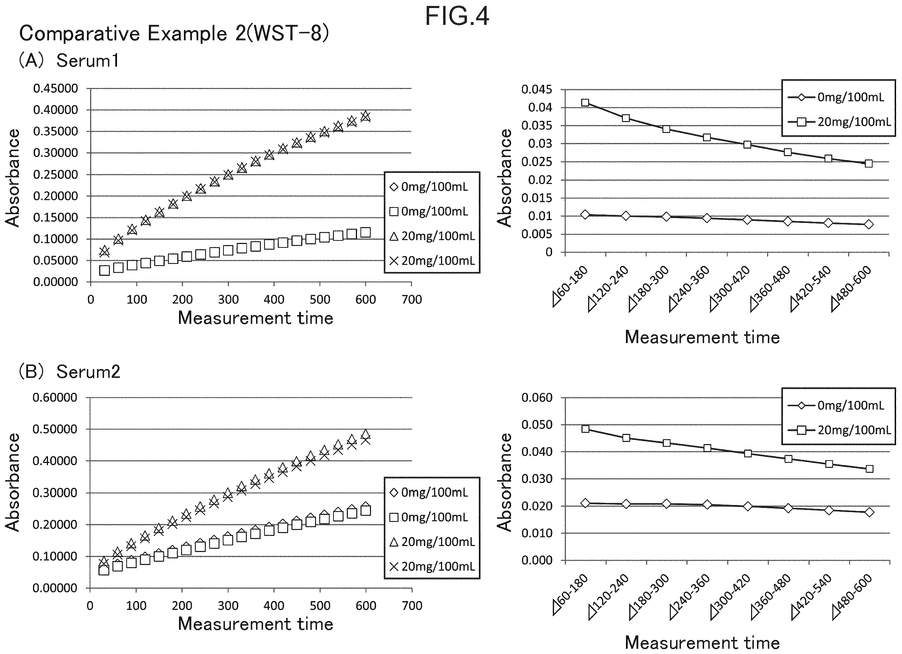

The results thereof are shown in FIGs. 2 to 4. FIGs. 2 to 4 are graphs showing the results of measuring the absorbance of the color reaction using each analysis test piece. FIG. 2 shows the results obtained by using the analysis test piece of Example 1, and FIGs. 3 and 4 show the results obtained by using the analysis test pieces of Comparative Examples 1 and 2, respectively. In each of FIGs. 2 to 4, the graphs on the left show the absorbance over time, and the graphs on the right show the change in absorbance per unit time.

In FIG. 2, (A) shows the results of analyzing the serum 1 using the analysis test piece of Example 1, and (B) shows the results of analyzing the serum 2 using the analysis test piece of Example 1. As shown in FIGs. 2(A) and (B), in Example 1 where WST-9 was used, even when uric acid was added, an increase in absorbance showing a high correlation with the lapse of time was confirmed from the graphs on the left, and the absence of fluctuations in the change in absorbance per unit time was confirmed from the graphs on the right, both in the same manner as in the case where no uric acid was added.

In FIG. 3, (A) shows the results of analyzing the serum 1 using the analysis test piece of Comparative Example 1, and (B) shows the results of analyzing the serum 3 using the analysis test piece of Comparative Example 1. Further, in FIG. 4, (A) shows the results of analyzing the serum 1 using the analysis test piece of Comparative Example 2, and (B) shows the results of analyzing the serum 2 using the analysis test piece of Comparative Example 2. As shown in FIGs. 3 and 4, it was confirmed that, from the graphs on the left, the serum samples with the addition of uric acid showed a higher absorbance than the serum samples without the addition of uric acid; and that, from the graphs on the right, the serum samples with the addition of uric acid showed greater fluctuations of decrease in the change in absorbance per unit time than the serum samples without the addition of uric acid.

From these results, it was found that, by using WST-9 as a color developer, a glycated protein can be analyzed with excellent accuracy without the effects of uric acid.

### [Example 2]

For the method of analyzing a glycated protein according to the disclosure, it was confirmed by measurement of fructosamine that the glycated protein concentration can be measured at a plurality of WST-9 concentrations without the effects of uric acid that is a reducing substance in blood.

As Example 2, six analysis test pieces 1 having different WST-9 concentrations were prepared in the same manner as in Example 1(2), except that the concentration of the color developer WST-9 in the second liquid reagent was changed to 1 mmol/L, 2.5 mmol/L, 5 mmol/L, 7.5 mol/L, 10 mmol/L and 20 mmol/L, respectively. Further, as serum samples, a serum 4 (without addition of uric acid (-)) having a fructosamine concentration of 220.3 µmol/L and a serum 5 (without addition of uric acid (-)) having a fructosamine concentration of 486.5 µmol/L, as well as a serum 4 (with addition of uric acid (+)) and a serum 5 (with addition of uric acid (+)) which were obtained by adding uric acid to the serum 4 (without addition of uric acid (-)) and the serum 5 (without addition of uric acid (-)) at a concentration of 20 mg/100 mL, respectively, were prepared. Then, using the respective six analysis test pieces 1 having different WST-9 concentrations and the thus prepared serum samples, the measurement of the absorbance was performed in the same manner as in Example 1.

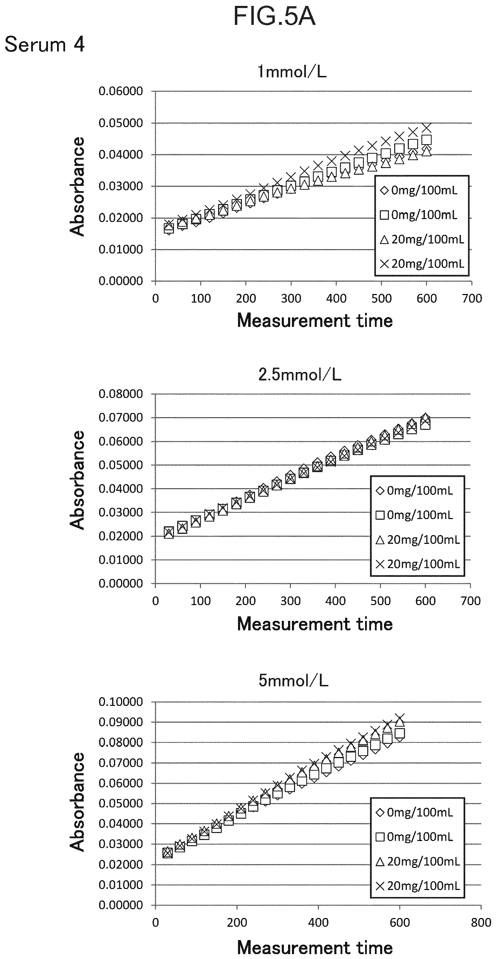

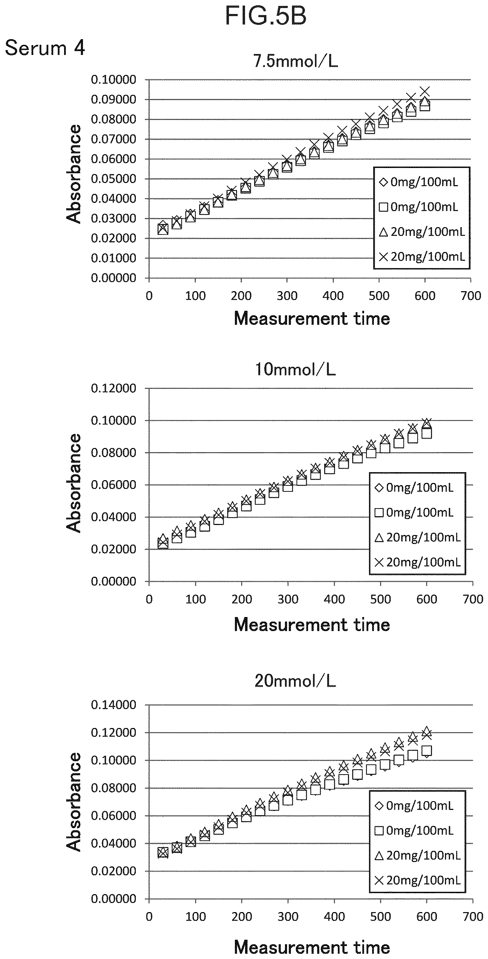

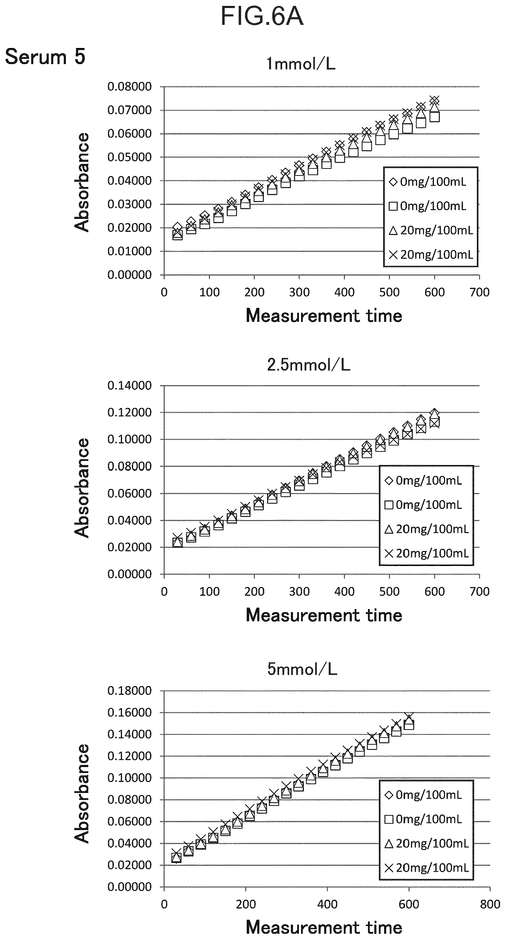

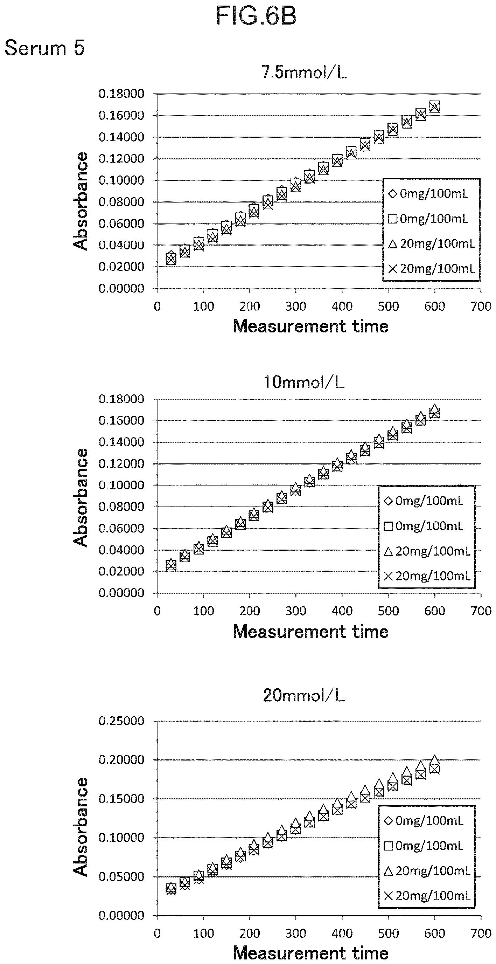

The results thereof are shown in FIGs. 5 and 6. FIGs. 5A and 5B show the absorbance over time at the respective WST-9 concentrations for the serum 4, and FIGs. 6A and 6B show the absorbance over time at the respective WST-9 concentrations for the serum 5.

As shown FIGs. 5A, 5B, 6A and 6B, even when uric acid was added, an increase in absorbance showing a high correlation with the lapse of time was confirmed at all WST-9 concentrations in the same manner as in the case where no uric acid was added.

From these results, it was found that the fructosamine concentration can be measured at a plurality of WST-9 concentrations without the effects of uric acid.

In a similar theory, a concentration of glycoproteins such as a glycated albumin or a glycated hemoglobin can be measured without the effects of uric acid that is a reducing substance in blood.

### INDUSTRIAL APPLICABILITY

As described above, according to the disclosure, by incorporating the above-described color developer, the glycated protein concentration can be measured without the effects of uric acid that is a reducing substance in blood. Therefore, the disclosure is said to be extremely useful in the fields of, medicines, diagnostic agents, measurement and the like.

## Claims

1. A method of analyzing a fructosamine, the method comprising:
reacting a color developer represented by the following Formula (1) with a biological sample; and
measuring a color reaction of the color developer, 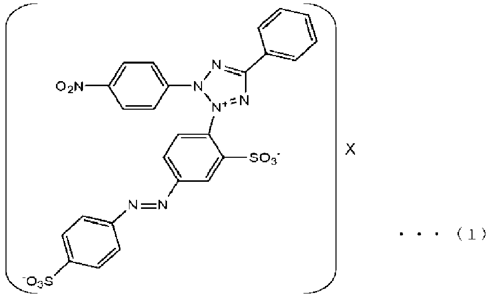
wherein, in Formula (1), X is an alkali metal ion.

2. The method according to claim 1, wherein the biological sample is a blood sample.

3. The method according to claim 2, wherein the blood sample is a serum sample or a plasma sample.

4. The method according to any one of claims 1 to 3, wherein the biological sample in a liquid state is added to the color developer in a dry state.

5. A use of a reagent for analyzing a fructosamine, in a method according to any of claims 1 to 4, the reagent comprising a color developer represented by the following Formula (1), 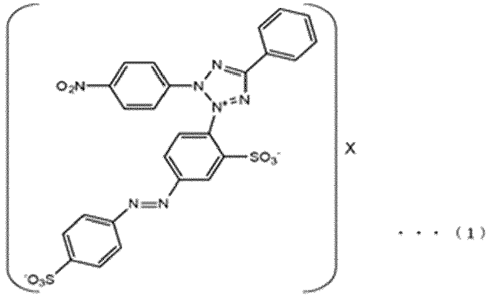 wherein, in Formula (1), X is an alkali metal ion.

6. A use of a kit for analyzing a fructosamine, in a method according to any of claims 1 to 4, the kit comprising a reagent which comprises a color developer represented by the following Formula (1), 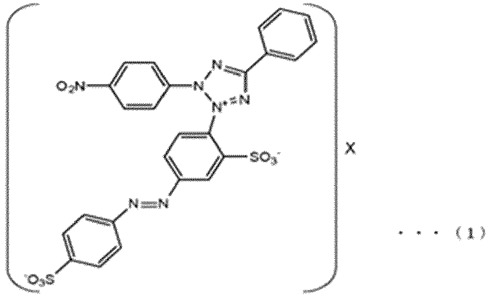 wherein, in Formula (1), X is an alkali metal ion.

7. A use of a test piece for analyzing a fructosamine, in a method according to any of claims 1 to 4, the test piece comprising a substrate,
wherein the substrate comprises a sample supply region and a reaction region, the sample supply region and the reaction region are connected by a flow channel, a reagent for analyzing a fructosamine is placed at the reaction region, and the reaction region in the substrate is composed of a transparent member, and
wherein the reagent comprises a color developer represented by the following Formula (1), 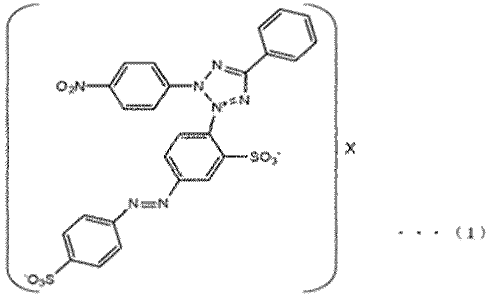 wherein, in Formula (1), X is an alkali metal ion.

8. A use of the test piece according to claim 7,
wherein the substrate is a layered body comprising an upper substrate and a lower substrate, the inside of the layered body comprises a first space, a second space, and the flow channel connecting the first space and the second space, the upper substrate comprises an opening at a position corresponding to the first space, the opening of the upper substrate and the first space form the sample supply region, the opening is a sample inlet, the second space is the reaction region, and the reagent is placed in at least a region of the upper substrate or a region of the lower substrate, the regions corresponding to the second space.

## Patentansprüche

1. Verfahren zum Analysieren eines Fructosamins, wobei das Verfahren umfasst:
Reagieren eines Farbentwicklers, der durch die folgende Formel (1) dargestellt ist, mit einer biologischen Probe; und
Messen einer Farbreaktion des Farbentwicklers, 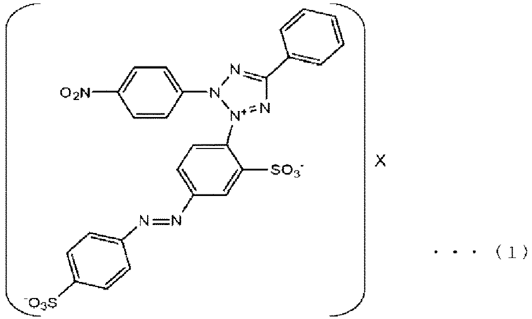 wobei in Formel (1) X ein Alkalimetallion ist.

2. Verfahren nach Anspruch 1, wobei die biologische Probe eine Blutprobe ist.

3. Verfahren nach Anspruch 2, wobei die Blutprobe eine Serumprobe oder eine Plasmaprobe ist.

4. Verfahren nach einem der Ansprüche 1 bis 3, wobei die biologische Probe in einem flüssigen Zustand dem Farbentwickler in einem trockenen Zustand zugegeben wird.

5. Verwendung eines Reagenz zum Analysieren eines Fructosamins in einem Verfahren nach einem der Ansprüche 1 bis 4, wobei das Reagenz einen Farbentwickler umfasst, der durch die folgende Formel (1) dargestellt ist, 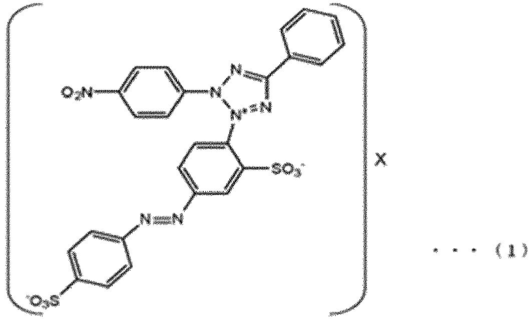 wobei in Formel (1) X ein Alkalimetallion ist.

6. Verwendung eines Kits zum Analysieren eines Fructosamins in einem Verfahren nach einem der Ansprüche 1 bis 4, wobei das Kit ein Reagenz umfasst, das einen Farbentwickler umfasst, der durch die folgende Formel (1) dargestellt ist, 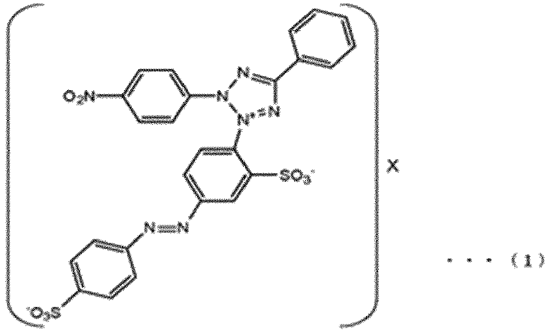 wobei in Formel (1) X ein Alkalimetallion ist.

7. Verwendung eines Teststücks zum Analysieren eines Fructosamins in einem Verfahren nach einem der Ansprüche 1 bis 4, wobei das Teststück ein Substrat umfasst,
wobei das Substrat einen Probenzuführungsbereich und einen Reaktionsbereich umfasst, der Probenzuführungsbereich und der Reaktionsbereich durch einen Strömungskanal verbunden sind, ein Reagenz zum Analysieren eines Fructosamins am Reaktionsbereich positioniert ist und der Reaktionsbereich im Substrat aus einem transparenten Element besteht, und
wobei das Reagenz einen Farbentwickler umfasst, der durch die folgende Formel (1) dargestellt ist, 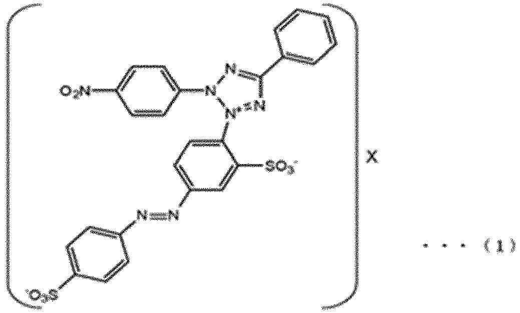 wobei in Formel (1) X ein Alkalimetallion ist.

8. Verwendung des Teststücks nach Anspruch 7,
wobei das Substrat ein Schichtkörper ist, der ein oberes Substrat und ein unteres Substrat umfasst, das Innere des Schichtkörpers einen ersten Raum, einen zweiten Raum und den Strömungskanal umfasst, der den ersten Raum und den zweiten Raum verbindet, das obere Substrat eine Öffnung an einer Position umfasst, die dem ersten Raum entspricht, die Öffnung des oberen Substrats und der erste Raum den Probenzuführungsbereich bilden, die Öffnung ein Probeneinlass ist, der zweite Raum der Reaktionsbereich ist und das Reagenz in mindestens einem Bereich des oberen Substrats oder einem Bereich des unteren Substrats positioniert ist, wobei die Bereiche dem zweiten Raum entsprechen.

## Revendications

1. Procédé d'analyse d'une fructosamine, le procédé comprenant :
la réaction d'un développeur de couleur représenté par la Formule (1) suivante avec un échantillon biologique ; et
la mesure d'une réaction colorée du développeur de couleur, 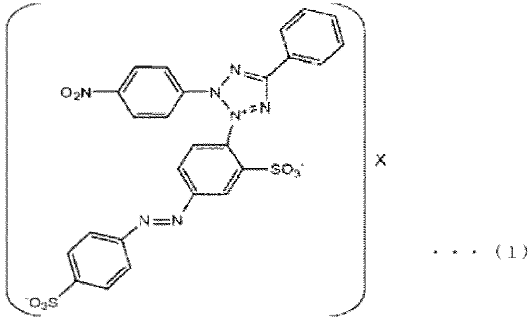 dans lequel, dans la Formule (1), X est un ion de métal alcalin.

2. Procédé selon la revendication 1, dans lequel l'échantillon biologique est un échantillon de sang.

3. Procédé selon la revendication 2, dans lequel l'échantillon de sang est un échantillon de sérum ou un échantillon de plasma.

4. Procédé selon l'une quelconque des revendications 1 à 3, dans lequel l'échantillon biologique dans un état liquide est ajouté au développeur de couleur dans un état sec.

5. Utilisation d'un réactif pour analyser une fructosamine, dans un procédé selon l'une quelconque des revendications 1 à 4, le réactif comprenant un développeur de couleur représenté par la Formule (1) suivante, 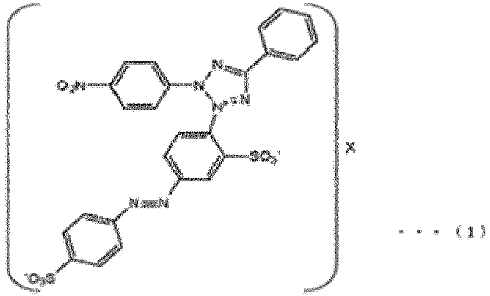 dans laquelle, dans la Formule (1), X est un ion de métal alcalin.

6. Utilisation d'un kit pour analyser une fructosamine, dans un procédé selon l'une quelconque des revendications 1 à 4, le kit comprenant un réactif qui comprend un développeur de couleur représenté par la Formule (1) suivante, 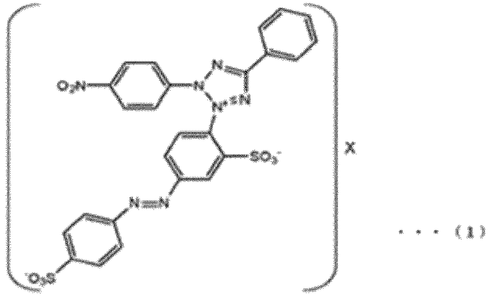 dans laquelle, dans la Formule (1), X est un ion de métal alcalin.

7. Utilisation d'une pièce de test pour analyser une fructosamine, dans un procédé selon l'une quelconque des revendications 1 à 4, la pièce de test comprenant un substrat,
dans laquelle le substrat comprend une région de fourniture d'échantillon et une région de réaction, la région de fourniture d'échantillon et la région de réaction sont reliées par un canal d'écoulement, un réactif pour analyser une fructosamine est placé au niveau de la région de réaction, et la région de réaction dans le substrat est composée d'un élément transparent, et
dans laquelle le réactif comprend un développeur de couleur représenté par la Formule (1) suivante, 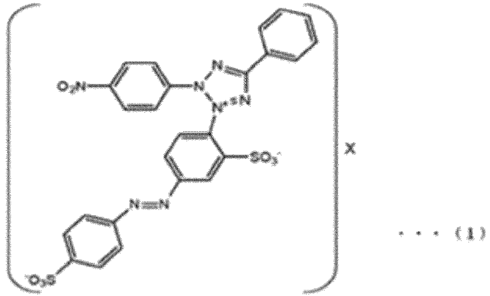 dans laquelle, dans la Formule (1), X est un ion de métal alcalin.

8. Utilisation de la pièce de test selon la revendication 7,
dans laquelle le substrat est un corps feuilleté comprenant un substrat supérieur et un substrat inférieur, l'intérieur du corps feuilleté comprend un premier espace, un second espace, et le canal d'écoulement reliant le premier espace et le second espace, le substrat supérieur comprend une ouverture à une position correspondant au premier espace, l'ouverture du substrat supérieur et le premier espace forment la région de fourniture d'échantillon, l'ouverture est une entrée d'échantillon, le second espace est la région de réaction, et le réactif est placé au moins au niveau d'une région du substrat supérieur ou d'une région du substrat inférieur, les régions correspondant au second espace.
